# EUROPEAN PATENT SPECIFICATION

(11) **EP 0 361 719 B1**
(45) Date of publication and mention of the grant of the patent: **12.01.1994**
(21) Application number: 89309169.4
(22) Date of filing: 08.09.1989
(51) Int. Cl.: A61K 37/14

(54) **Haemoglobin based blood substitute and method for the preparation thereof**
Blutersatzmittel mit Hämoglobinen als Grundstoff und Verfahren zur Herstellung
Substitut du sang à base d'hémoglobine et méthode de préparation

(30) Priority: 08.09.1988 IL 87707
(43) Date of publication of application: 04.04.1990
(73) Proprietor: Technion Research & Development Foundation Ltd., Haifa 32000 (IL)
(72) Inventor: Ilan, Ehud, IL-62598 Tel-Aviv (IL); Sideman, Samuel, Haifa (IL); Lotan, Noah, Haifa (IL); Cohen, Tova, IL-20100 Carmiel (IL)
(74) Representative: Clifford, Frederick Alan

(56) References cited:
- WO-A-87/07832
- WO-A-88/03408
- US-A- 4 584 130
- A.S.A.I.O. TRANSACTIONS (AMERICAN SOCIETY FOR ARTIFICIAL INTERNAL ORGANS), vol. 33, no. 4, December 1987, pages 819-823, Hagerstown, MD, US; T.M.S. CHANG et al.: "Blood substitutes based on modified hemoglobin and fluorochemicals"
- INT. J. ARTRITIC ORGANS, vol. 6, no. 6, 1983, pages 319-320; R. STABILINI et al.: "A pyridoxylated polymerized hemoglobin solution as oxygen carrying substitute"
- APPL. BIOCHEM. BIOTECHNOL., vol. 10, 1984, pages 113-141, The Humana Press Inc.; P.E. KEIPERT et al.: "Preparation and in vitro characteristics of a blood substitute based on pyridoxylated polyhemoglobin"
- TRANSFUSION, vol. 23, no. 2, 1983, pages 158-162, J.B. LIPPINCOTT CO.; I.R. SEHGAI et al.: "Preparation and in vitro characteristics of polymerized pyridoxylated hemoglobin"

## Description

The present invention relates to blood substitute material. More particularly, the invention relates to a hemoglobin-based blood substitute material and to a method for the preparation thereof.

As known, blood transfusion has become a universally accepted, life-saving procedure used in modern clinical medicine. Blood losses at substantial amounts are very dangerous, since they decrease the blood volume and pressure, limit the oxygen supply to vital tissues and slow the metabolic processes. However, the collection of blood for transfusion, its storage and administration is a complex subject which involves serious problems such as limited resources, compatibility and risk of transmitting certain diseases (e.g. hepatitis, AIDS, malaria, etc.).

Moreover, when considering performing the blood transfusion in the field, even more complicated problems are encountered, not only of medical origin but also logistic, such as availability at site, transport and storage conditions, etc.

In view of she above problems, the search for blood substitutes was seriously considered and a number of related paper and patents can be found in the respective literature.

Two main groups of blood substitutes are mentioned in the literature:(a) perfluorocarbons-based, and (b) hemoglobin-based. Perfluorocarbons-based substitutes are cyclic -, branched, or straight-chain molecules, in which hydrogen atoms have been replaced with fluorine. Due to the strength of the carbon-fluorine bond, these compounds are generally considered as very inert, both chemically and biologically. They are capable of absorbing oxygen by physical dissolution, the amount absorbed being linearly dependent on the composition of the equilibrating gas phase. However, recent studies have shown that perfluorocarbons have several disadvantages such as adverse reactions on the immune system, hematological disturbances, alterations in protaglandin metabolism, impairment of the coagulation process, etc.

The present invention relates to a hemoglobin-based substitute, and therefore some more discussion will be presented on this subject. As known, hemoglobin binds oxygen in a process having the characteristic of non-linearity, and a chemical species known as oxyhemoglobin is thus formed. An ideal hemoglobin-based blood substitute should have the ability to transport and supply oxygen from the lungs to vital tissues and organs, and to transport carbon dioxide from these tissues and organs back to the lungs. In the same time, it must have minimal side effects, so that it could be tolerated in large doses.

It is also known that hemoglobin is a tetrameric protein, composed of equal numbers of two different protein chains, alpha and beta. Under physiological conditions, the predominant molecular species is the said tetramer, having a molecular weight of about 65,000. In mammals, hemoglobin is present in erythrocytes, the membrane of which contains stroma which consists of phospholipids, cholesterol, and proteins.

In the followings, the terms stroma-free hemoglobin and stroma-depleted hemoglobin can be used interchangeably, and both mean a product from which essentially all the stromal components were removed.

Attempts have been made to use stroma-free hemoglobin solutions as blood substitute, exhibiting oxygen transport capabilities. These solutions endure storage at room temperature for limited periods of time, without changes in their properties. However, oxygen binds to hemoglobin very strongly, so that only a small part of the bound oxygen is released to the cells at the oxygen tension normally prevailing in tissues. Also, not all of the important characteristics of these solutions are equal to those of human blood. Thus for instance, at the required concentration, the corresponding oncotic pressure is far from that of normal blood. Further investigations have also shown that stroma is a toxic factor, probably by causing thrombosis of the small renal vasculature.

In view of some favorable properties possessed by stroma-free hemoglobin, intensive work was done to improve the disadvantages encountered with its use as blood substitute. One approach was to modify its structure in order to decrease its oxygen affinity. For this purpose, it was suggested by Benesch et al [Biochemistry 11, 3576-3582 (1972)] to treat the stroma-free hemoglobin with pyridoxal-5′-phosphate (hereafter referred to as PLP).

The product obtained, pyridoxal-5′-phosphate hemoglobin (hereafter referred to as PLP-hemoglobin), indeed has a satisfactory oxygen affinity, but the intravascular life time is too short compared to what is required for a resuscitation material.

Based on the same approach of modifying the structure of stroma-free hemoglobin, it is described in the U.S. Patent Number 4,061,736 a method for obtaining an intramolecularly crosslinked stroma-free hemoglobin, by reacting the latter with a reagent selected from aldehydes, dialdehydes, activated ureas, derivatives of carboxylic acids, heterocyclic triazines and halogenated aromatic cycloalkanes.

According to U.S. Patent No. 4,598,064, stroma-free hemoglobin is modified by intramolecular crosslinking between the alpha chains of the tetramer, using an acylating agent. It is claimed that the product obtained is an effective blood substitute having suitable oxygen binding characteristics, such as low oxygen affinity, being particularly useful in the treatment of ischemia.

Another approach, which was also investigated, consists in the modification of hemoglobin in an attempt to improve both the oxygen binding characteristics and the intravascular retention. Thus according to PCT Patent Application WO 84/04248, hemoglobin is first covalently crosslinked intramolecularly between the two beta chains, and then reacted with PLP. It is claimed that the product thus obtained is a stable, oxygen carrying material, capable of delivering oxygen to perfused tissue, and advantageously remaining in the intravascular space.

However, one of the main disadvantages of the blood substitute obtained according to the method described therein is its limited oxygen carrying capacity.

Still another approach involves the polymerization of PLP-hemoglobin [G.S. Moss et al, Surgery 95, 249-255 (1984)]. However, the product thus obtained has, again, a limited oxygen carrying capacity.

Summing-up the above short review of the prior art, it should be concluded that there are, indeed, some improvements towards the use of hemoglobin-based blood substitutes beyond the use of stroma-free hemoglobin.

Blood substitutes containing one or more characteristics of normal blood can thus be encountered, but still there is a long felt need for a blood substitute which will possess most of the characteristics of the normal blood.

It is an object of the present invention to provide a blood substitute which possesses most of the characteristics of the normal blood. It is another object of the present invention to provide a hemoglobin-based blood substitute which possesses most of the characteristics of the normal blood. It is yet another object of the present invention to provide a hemoglobin-based blood substitute to be useful for clinical use. A further object of the present invention is to provide a simple method for obtaining a hemoglobin-based blood substitute useful for clinical use.

The invention consists in hemoglobin-based blood substitute resulted from stroma-free hemoglobin, modified with pyridoxal-5'-phosphate and intramolecularly stabilized, followed by the polymerization to an extent of above 40%, under anaerobic conditions of the stabilized PLP-hemoglobin, characterized in that the intramolecular stabilization is effected by reaction with a haloester of a dicarboxylic acid, said blood substitute material possessing a hemoglobin concentration in the range of 12 to 10 g/100 ml, possessing a P₅₀ in the range of 25 to 30 torr (25 to 30 mm Hg), and possessing a colloid osmotic pressure in the range of 24 to 31 torr (24 to 31 mm Hg).

The product obtained, when reconstituted with an appropriate solvent, yields a blood substitute solution which is characterized by properties which are substantially equal to those of whole human blood, and which are highly improved when compared to those of solutions of unmodified hemoglobin. Table 1 below summarizes the main properties of whole human blood under normal physiological conditions, of a solution of unmodified hemoglobin [the values for unmodified hemoglobin solution were taken from Lakshman et al., Journal of Surgical Research 30, 14-20 (1981)], and of a solution of the hemoglobin-based blood substitute obtained according to the present invention.

**TABLE 1**

| Main properties of whole human blood, of a solution of unmodified hemoglobin and of a solution of the blood substitute material prepared according to the present invention | | | |
|---|---|---|---|
| Property | Whole human blood | Solution of Unmodified Hemoglobin | Solution of Blood Substitute |
| - Hemoglobin conc. (g/100 ml) | 12-18 | 7- 8 | 10-18 |
| - P₅₀ (torr) (mm Hg) | 26-29 | 12-14 | 25-30 |
| - COP (torr) (mm Hg) | 25-29 | 20-25 | 24-31 |
| - Methemoglobin content(% of total Hb) | up to 5 | 3-8 | 3-6 |
| P₅₀ = the partial pressure of oxygen at which 50% of hemoglobin sites are saturated with oxygen; measured at normal physiological conditions: pH = 7.4, pCO₂ = 40 torr (40 mm Hg), temperature = 37.0°C. COP = colloid osmotic (i.e. oncotic) pressure. | | | |

As appears from the above Table 1, a solution of the blood substitute material prepared according to the present invention is characterised by its proper oxygen affinity, as well as by its adequate COP at normal physiological concentrations of hemoglobin.

The blood substitute prepared according to the present invention is characterized by the following advantageous properties:
a. Is not toxic.
b. Possesses appropriate lifetime in circulation.
c. The solution obtained upon reconstitution in an appropriate solvent possesses good oxygen binding and delivery characteristics.
d. The solution obtained upon reconstitution in an apropriate solvent possesses normal oncotic pressure at physiological concentrations of hemoglobin, and correspondingly appropriate oxygen transport capacity.

In view of the above, transfusion with this blood substitute solution is much more convenient to perform than transfusion with whole blood, because there is no need to "type and crossmatch" prior to transfusion and thus lives may be saved by saving precious time.

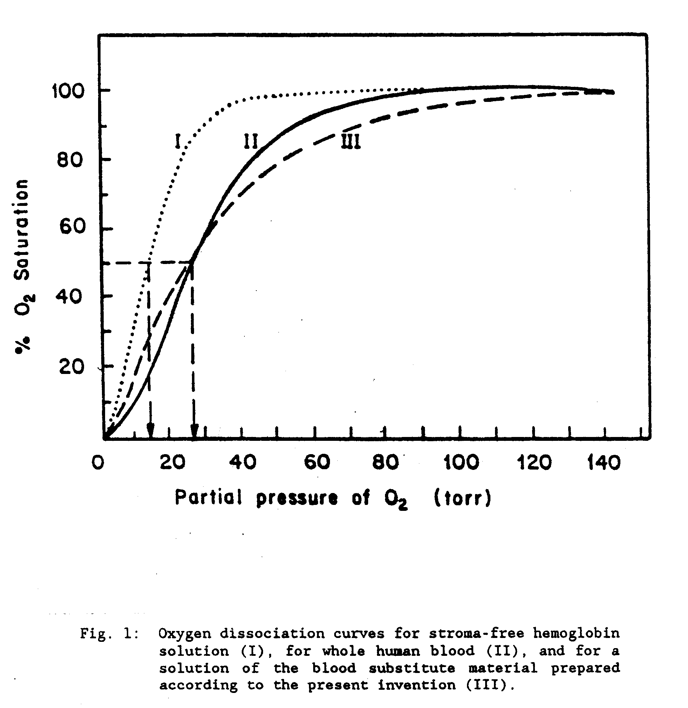

One of the important properties required for a blood substitute is to have an appropriate oxygen dissociation curve. In Figure 1 there is a diagram illustrating in a clear manner this property for whole human blood (II), for the solution of the blood substitute prepared accordding to the present invention (III), and for the solution of stroma-free hemoglobin (I), which is the starting material for said blood substitute material. As appears from Figure 1, the P₅₀ of the blood substitute solution (25-30 torr) (25-30 mm Hg) is virtually equal to that of whole human blood (26-29 torr) (26-29 mm Hg) whereas the P₅₀ of stroma-free hemoglobin solution (12-14 torr) (12-14 mm Hg) is very much lower than that of whole blood.

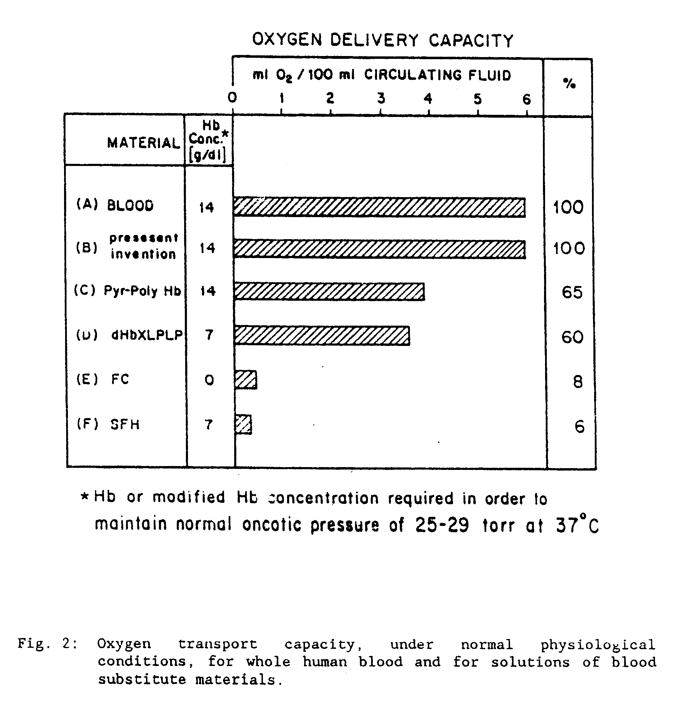

Another important property required for a blood substitute is to have the appropriate capacity for carrying oxygen from the lungs to the tissues. This characteristic is presented schematically in Figure 2, for whole human blood (A),for a solution of the blood-substitute prepared according to the present invention (B), for the blood-substitute solutions described in the prior art and obtained from polymerized PLP-hemoblobin (C) [G. S. Moss et al., Surgery 95, 249-255 (1984)] and from intramolecularly crosslinked pyridoxylated hemoglobin (D) (PCT Pat. Application WO 84/04248), for Fluosol DA fluorocarbon emulsion (E) [H. Ohyanagi and Y. Saitoh, Int. J. Art. Organs 9, 363-368 (1986)] and for stroma-free hemoglobin solution exhibiting the required oncotic pressure (F). As appears from this Figure, the oxygen carrying capacity - measured relatively to whole human blood at normal physiological conditions - is 100% for the blood substitute prepared according to the present invention, compared wih 65% or 60% for the prior art blood substitute solutions, 8% for fluorocarbons, and 6% for the stroma-free hemoglobin solution.

The hemoglobin - based blood substitute material prepared according to the present invention is much superior to the perfluoro-chemicals suggested as blood substitutes in casualties suffering from hypovolemic shocks, due to its ability to carry appropriate amounts of oxygen when the victim is ventilated with room air. It may also be used to treat acute myocardial infarcts, and cerebral vascular accidents.

The improved properties of the blood-substitute prepared according to the present invention result from the polymerization of the intramolecularly stabilized PLP-hemoglobin. It was found that this polymerization should be carried out only in absence of oxygen, and under conditions in which the percentage of polymerization is at least above 40% and preferably in the range of 50% to 80%.

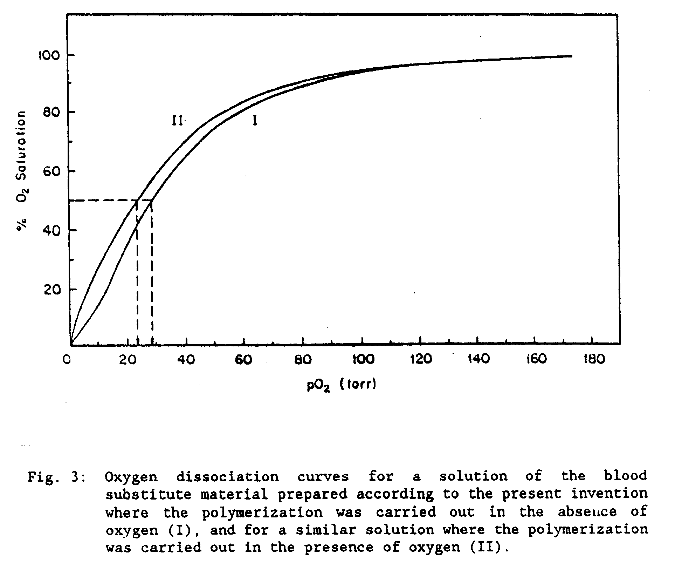

In Figure 3 are presented two graphs which correlate the percentage of oxygen saturation and the partial pressure of oxygen (expressed in torr (mm Hg)). Graph I is the oxygen dissociation curve of a solution of the blood substitute prepared according to the present invention, particularly in case that the said polymerization is carried out in the absence of oxygen, whereas graph II when said polymerization is carried out in the presence of oxygen.

In the following Table 2 the main characteristic properties of the products obtained in the absence and presence of oxygen are presented.

**TABLE 2**

| Characteristic properties of solution of the blood substitute material prepared according to the present invention when the polymerization is carried out in the presence or absence of oxygen | | |
|---|---|---|
| Property | Product obtained in the absence of oxygen | Product obtained in the presence of oxygen |
| - Hemogl.conc. (g/100 ml) | 12.4 | 14.5 |
| - Methemoglobin content (% of total hemoglobin) | 5.5 | 16.9 |
| - P₅₀ (at pH = 7.4; pCO₂=40 torr (40 mm Hg) & T=37°C | 28 | 24 |

From the above data it clearly appears that the polymerization in the presence of oxygen leads to a product with a P₅₀ of 24 torr (24 mm Hg), which is significantly lower than that of the corresponding product obtained by polymerization in the absence of oxygen (P₅₀ = 28 torr (28 mm Hg)). Also, the methemoglobin content in the product is much higher when the polymerization is carried out in the presence of oxygen rather than in its absence (16.9% and 5.5%, respectively).

Polymerization reagents for this step should be selected from various compounds such as glutaraldehyde or succinic dialdehyde or precursors thereof such as 2-ethoxy-3,4-dihydro-1,2-pyran, 2,5-diethoxy tetrahydrofuran, 2-amino-4,6-dichloro-S-triazine, bis-diazobenzidine 2,2′-sulfonic acid, 1,5-difluoro-2,4-dinitrobenzene, ethylene glycol diglycidyl ether, butadiene diepoxide etc.

Generally, the polymerization reagent is added to a buffered solution of stabilized PLP-hemoglobin having a pH between 6.5 and 8.5, and preferably of 7.5.

According to the most preferred embodiment, the polymerized product obtained is quenched in an amine solution in order to avoid that moieties containing active groups of the polymerization reagent remain in the final product.

Accordingly, the amine should be added in an amount sufficient to quench all the unreacted groups of the polymerization reagent. Although amines such as ethanolamine and lysine are preferred reagents, other reagents may be utilized, such as bisulfites, diols, alcohols, isocyanates and mercaptans.

An additional optional step which, if carried out anaerobically, would avoid an increase in the methemoglobin content, is reduction with sodium borohydride. This step ensures the saturation of the double bonds formed by the polymerization reagent, thus giving extra stability to the polymerized hemoglobin. Since, at the introduction of sodium borohydride, apart from the reduction of the double bonds, the residual carbonyl groups introduced by the polymerization reagent are also reduced, the addition of a quenching agent is generally superfluous. In most cases, only one additional step after the polymerization, i.e. quenching or reduction with borohydride will be indicated, but for specific cases such as when extra stability of the polymerized product is needed together with precise regulation of the polymerization step, the two additional steps might be indicated. A person skilled in the art, will select the proper step according to the specific requirement for the blood-substitute.

One of the advantages of the process for obtaining the hemogobin-based substitute according to the present invention, is the use of common reagents, commercially available or easily synthesized, which are generally utilized for this purpose. The product obtained, may be lyophilized in the presence of 3% glucose or other protective agents as known in the art, without changing its oxygen - transporting properties as a potential resuscitation fluid upon reconstitution.

It is important to note that only a blood substitute solution prepared according to the present invention possess all the crucial characteristics of normal blood, as shown in Table 1 and Figures I and II of this procedure. Other products as described in the literature, prepared by various methods even though obtained using glutaraldehyde, for example, lack at least one critical property of normal blood. For example, this is the case for deoxyhemoglobin polymerized with glutaraldehyde (U.S. Patent No. 4,053,590 Example II), and for pyridoxylated glutaraldehyde-polymerized human deoxyhemoglobin [F. De Venuto et al., Journal of Surgical Research 34, 205-212 (1983)].

In summary, the blood-substitute hemoglobin-based material prepared according to the present invention is a superior product for the rapid treatment of hypovolemic shock, because it can be used by paramedical personnel without delay. It is easy to prepare, has a long storage life and delivers oxygen to perfused tissues when the victim is inhaling room air. It may be used in many critical situations other than combat casualty and hypovolemic shock. It should be an ideal substitute of whole blood for use as priming fluid in extracorporeal pumps employed in cardiac surgery, or in extracorporeal devices employed in blood treatments such as hemoperfusion and hemodialysis. It also could be used to treat ischemic episodes including sludging and pain in sickle cell crises. It could be used as an organ perfusant to maintain organ viability until transplanted.

### DETAILED DESCRIPTION OF THE INVENTION

The first step requires the obtaining of stroma-free hemoglobin. This is carried out starting with packed red blood cells or whole blood from various mammalian species species. There are several known methods for obtaining the stroma-free hemoglobin, most of them producing a suitable starting material for the present invention. As known, when stroma is present in hemoglobin preparations in large amounts, it interferes with renal function.

Investigations in the past had shown that stroma caused thrombosis of the small renal vasculature. It was already described in many references that by removal of most of the stroma from hemoglobin solution, this disadvantage is eliminated.

The method selected in the present invention for preparing the stroma-free hemoglobin is fully described in a paper by De Venuto et al. [J. Lab. Clin. Med. 89, 509- 516 (1977)].

In the second step, the stroma-free hemoglobin is pyridoxylated using a method similar to the one described by Benesch et al [Biochemistry 11, 3576-3582 (1972)].

As mentioned in the literature, the resulting solution of PLP-hemoglobin exhibits a P₅₀ of 20-24 torr (20-24 mm Hg), at normal physiological conditions, compared with 12-14 torr (12-14 mm Hg) shown by a corresponding solution of unmodified hemoglobin.

The third step involves the intramolecular stabilization of the PLP-hemoglobin. This is carried out by a chemical reaction with a compound selected from halo-esters of dicarboxylic acids, such as bis(3,5-dibromosalicyl) glutarate; bis(3,5-dibromosalicyl) adipate, bis(3,5-dibromosalicyl)fumarate; bis(3,5-dichlorosalicyl) fumarate; bis(3,5-diiodosalicyl)fumarate; bis(3,5-dibromosalicyl) succinate. The reaction introduces a covalently bound glutarate (or adipate, succinate, fumarate etc.) bridge between the two beta chains of the hemoglobin molecule.

This modification brings about an improved intravascular retention and oxygen transport capability of the product compared to the PLP-hemoglobin solution. The P₅₀, at normal physiological conditions, of the solution after this intramolecular stabilization increases to 28-31 torr (28-31 mm Hg), a value which is even higher than the normal P₅₀ of whole human blood. There are several variations for carrying out this reaction, but in particular suitable for the present invention was the route described by R. W. Tye et al. (Advances in Blood Substitute Research, R. B. Bolin, R.P. Geyer & G.J. Nemo eds., Alan R .Liss Inc. 1983, pages 41-49).

The fourth step is the crucial one for obtaining the blood substitute with the improved life time in circulation and adequate oncotic pressure. This step involves the polymerization under anaerobic conditions of the stabilised PLP-hemoglobin obtained in the previous step, at an extent of above 40% and most preferably at an extent in the range of between 50% and 80%. This polymerization is carried out using polyfunctional agents having groups that are capable of reacting with amino groups and other linkable sites on the hemoglobin molecule. In particular useful is the reaction with bi-or polfunctional carbonyl-containing compounds. Within this group of materials, glutaraldehyde was found to produce excellent results. It was found that the preferred molar ratio of polymerizing reagent - to hemoglobin ranges between 1:1 to 30:1. The oxygen-free environment, required in this step is maintained by continuously bubbling a stream of inert gas such as nitrogen.

Preferably, the reaction is carried out on a stabilized PLP-hemoglobin solution having a hemoglobin concentration of between 7 to 16% w/v. In order to prevent foaming during the reaction, addition of an antifoaming reagent is suggested. Optionally, at the end of this step, the product may be quenched and/or reduced with sodium borohydride in order to improve its qualities (see also: Brief Description of the Invention).

The obtained product possesses the properties desired for a blood-substitute.

The invention will be further illustrated by the following Examples without being limited thereto. It should be pointed out that Example IV does not illustrate the invention and is presented only for comparison purposes.

### EXAMPLE I.

### STEP 1.

A solution of washed crystals of stroma-free hemoglobin was prepared as per the method of De Venuto et al.[J.Lab. Clin. Med. ₈₉ 509-516 (1977)] and was dialyzed at 4°C, first twice against distilled water (1:24 by volume) in order to reduce the potassium and phosphate content and then three times more (1:16 by volume) against Tris-HCl buffer (0.1 M. pH 7.4, also containing 0.2 g/l ascorbic acid and 1 g/l glucose). At the end of this procedure, the pH of the retenate solution was 7.4.

In order to ensure sterility to the resulted solution, sodium penicillin G, gentamycin and streptomycin sulfate were added to final concentrations of:
- 50,000 units/l sodium penicillin G.
- 50 mg/l gentamycin; and
- 50 mg/l streptomycin sulfate.

### STEP 2.

An amount of 500 ml of the stroma-free hemoglobin solution obtained in step 1, with a concentration of 15%-20% w/v introduced into a 1.5 l closed pyrex container together with 2.5 ml of caprylic alcohol as antifoaming agent. An oxygen-free environment was achieved by bubbling continuously a stream of nitrogen, and a deoxygenated solution was added consisting of PLP (10 moles/mole of hemoglobin) dissolved in a mixture of 50 ml Tris-HCl buffer (1.0 M pH 7.4) and 25 ml Tris-HCl buffer (1.0 M, pH 8.0). The reaction between hemoglobin and PLP was carried out under continuous nitrogen bubbling for about 1 hour at about 25°C. The resulting solution was then treated with 15 ml of deoxygenated sodium borohydride solution (20 moles/mole of hemoglobin) in 1mM NaOH. This reaction was carried out for two hours at 25°C under nitrogen bubbling.

### STEP 3.

In this step, there is an intramolecular stabilization of the PLP-hemoglobin product obtained in step 2. In the same container a deoxygenated solution was added, consisting of bis-(3,5-dibromosalicyl) fumarate (2 moles/mole hemoglobin) dissolved in 50 ml Tris-HCl buffer (0.5 M, pH 8.0). The reaction was carried out in the absence of oxygen, nitrogen gas being bubbled continuously for about two hours, at 35°C and the resulting mixture was twice centrifuged,each time for 30 minutes at 4°C and 12,000xg, in order to eliminate particulate matter. The clear solution thus obtained was subsequently dialyzed four times at 4°C each time against 16 volumes of a standard kidney dialysis fluid [for composition see: De Venuto et al., J. Lab. Clin. Med. 89, 509-516 (1977)] also containing 0.2 g/l ascorbic acid and 1.0 g/l glucose; the pH was adjusted to 7.0 with lactic acid. At the end of this procedure, the reagents for ensuring sterility were added to the retenate to reach the concentrations as mentioned in step 1 for the final stroma-free hemoglobin solution.

### STEP 4.

An amount of 500 ml solution of the intramolecularly stabilized, PLP-hemoglobin, obtained in step 3, and containing 15% w/v hemoglobin, was introduced into a 1.5 1 closed Pyrex container together with 2.5 ml of caprylic alcohol (as antifoaming agent). A continuous stream of nitrogen gas was bubbled through the solution for about 1.5 hours. To the resulted deoxygenated solution, 24 ml of a deoxygenated solution of glutaraldehyde (2.5% w/v) -i.e. 8mg of glutaraldehyde per 1.0 g of hemoglobin-, in a standard kidney dialysis fluid (pH 7.0), was slowly added, while the stream of nitrogen gas continued. The reaction was continued for about three hours at 25°C, still under nitrogen streaming obtaining the desired extent of polymerization in the range of 40% to 80%.

In order to ensure the elimination of the residual active groups, the product obtained above was quenched with 50 ml deoxygenated solution of 1.0 M ethanolamine in standard kidney dialysis fluid, the pH of which was adjusted to 7.5 with 6 M hydrochloric acid. After 1 hour quenching at 25°C, 15 ml of sodium borohydride solution (20 moles/mole of hemoglobin) in 1 mM NaOH were added anaerobically, and nitrogen gas bubbling into the solution was continued.

After 2 hours, the reaction was terminated by dialysis, at 4°C, four times (1:20 v/v each time) against a standard kidney dialysis fluid also containing 0.2 g/l ascorbic acid and 1 g/l glucose, the pH being adjusted to 7.4 with lactic acid. Analysis of the extent of polymerization was done by high performance gel permeation chromatography (HP-GPC) using the TSK G3000SW LKB column, and the resulting elution profile showed that the reaction resulted in 50% polymerization. The hemoglobin-based blood substitute solution was further treated (sterilization etc.) to obtain the transfusion solution as known in the art.

### EXAMPLE II.

Polymerization, under anaerobic conditions, of the intramolecularly stabilized PLP-hemoglobin with different concentrations of glutaraldehyde. The reaction was carried out following the procedure described in Example I, except that, instead of 8 mg of glutaraldehyde per 1.0 g of homoglobin, use was made of 10, 12 and 16 mg of glutaraldehyde per 1.0 g of hemoglobin. The reaction mixture which contained 16 mg glutaraldehyde per 1.0 g of hemoglobin became very viscous and approximately 5 min. after the addition of glutaraldehyde it turned into a gel which was quite difficult for handling. The products obtained by using 10 and 12 mg glutaraldehyde per 1.0 g hemoglobin showed properties as presented in Table 1 and Figures 1 and 2.

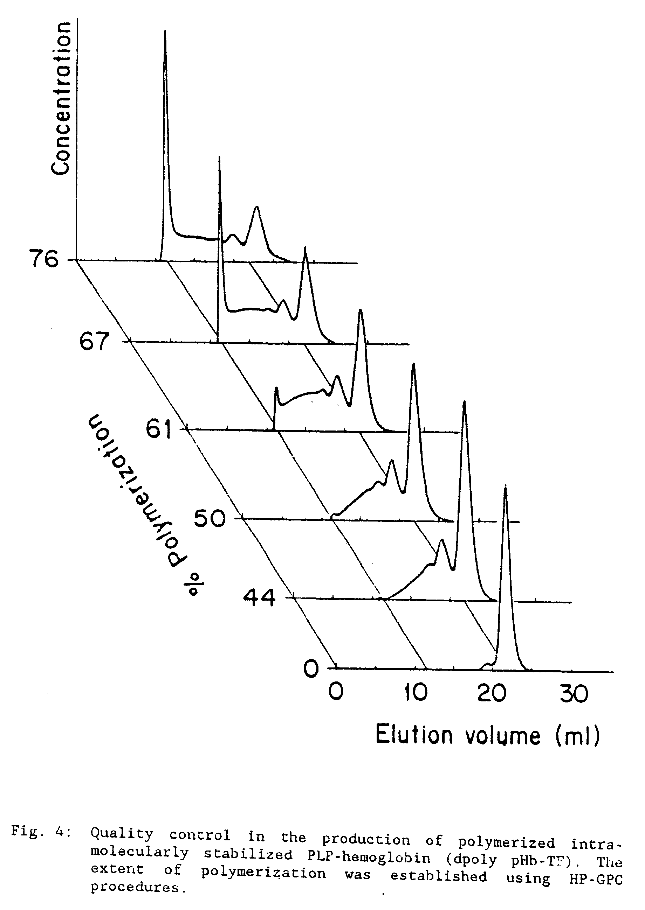

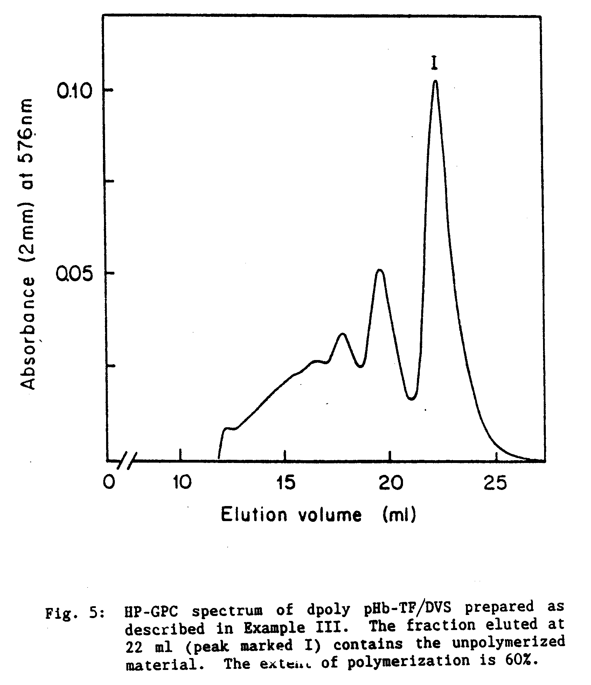

In the products described above, the extent of polymerization was determined by HP-GPC, and was found to be 67% and 76% for the products obtained at 10 and 12 mg glutaraldehyde per 1.0 g hemoglobin, respectively (see the attached Figure 4).

### EXAMPLE III.

Anaerobic polymerization of the intramolecularly stabilized PLP-hemoglobin, using divinylsulfone. As starting material for this reaction the solution obtained in Step 3 of Example I was used.

An amount of 130 ml solution, obtained in Step 3 of Example I and containing 16% wv hemoglobin, was introduced into a 1 l closed Pyrex container, together with 0.65 ml of caprylic alcohol. A continuous stream of nitrogen gas was bubbled through the solution for 75 minutes at 25°C. To the resulted deoxygenated solution, 0.7 ml of pure divinylsulfone was added dropwise, while the stream of nitrogen gas continued. The reaction was continued for three more hours at 25°C, still under nitrogen streaming. The product was quenched with 13 ml deoxygenated solution of 1.0 M ethanolamine in standard kidney dialysis fluid, the pH of which was adjusted to 7.5 with 6M hydrochloric acid. After 1 hour quenching at 25°C, 4 ml solution of sodium borohydride (20 moles/mole hemoglobin) in 1 mM NaOH were added anaerobically, and nitrogen gas bubbling into the solution was continued.

After 1.5 hours, the reaction was terminated by dialysis, at 4°C, four times (1:15 v/v each time) against a standard kidney dialysis fluid also containing 0.2 g/l ascorbic acid and 1 g/l glucose, the pH being adjusted to 7.4 with lactic acid. This hemoglobin-based blood substitute solution was further treated to obtain the transfusion solution as known in the art.

The final product thus obtained contains 13% w/v hemoglobin and 6% w/v methemoglobin. The P₅₀ was measured at 37°C under normal physiological conditions (pH 7.4, pCO₂ - 40 hour) and found to be 45 torr (45 mm Hg).

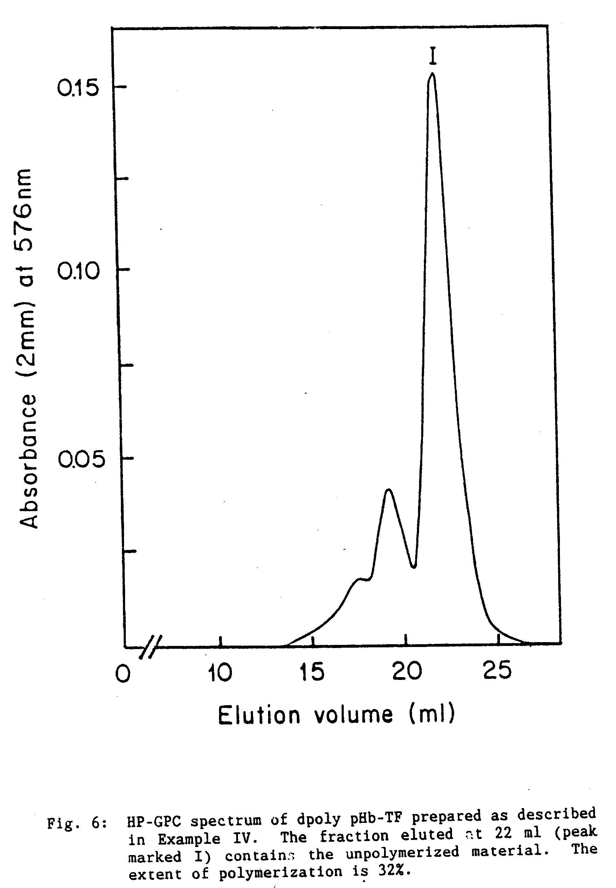

The product was analyzed by HP-GPC (see Figure 6), and its extent of polymerization was determined to be 60%.

The oncotic pressure of a 10% w/v solution of the final product was 28 torr (28 mm Hg).

### EXAMPLE IV. (comparative Example).

Pyridoxylation, intramolecular stabilization and polymerization, of stroma-free hemoglobin reaction were carried out essentially following the steps described in Example I under conditions which provide a polymerization extent of only 32%, as determined by the HP-GPC procedure (see Figure 6).

The resulted blood substitute upon reconstitution in an appropriate solvent, with a physiological hemoglobin concentration of 15% w/v, possessed an oncotic pressure of above 40 torr (40 mm Hg) which is much higher than that of whole human blood and therefore will not possess an appropriate oxygen transport capacity.

Preliminary toxicity tests were performed on six white mice with transfusion solution of the blood substitute material obtained according to the present invention.

Thus, an amount of 0.1 ml of the blood substitute solution containing 12.4 g/100 ml hemoglobin was injected through the tail of each mouse. Normal behaviour of the mice was noticed until they were sacrificed fifteen days after the transfusion.

Partial exchange transfusions (25-35% of blood volume) with solutions of the blood substitute material of this invention were performed in four rabbits. Exchange transfusion was done by removing 25 ml of blood from an artery in one ear and immediate infusion of 25 ml of blood substitute solution through a vein in the other ear, repeating the operation at 10 minutes intervals until the desired exchange level was achieved. The four rabbits receiving the blood substitute lived until they were sacrificed at 3-44 day for necropsy. No abnormal signs were found in histopathological tests of tissues from bladder, heart, kidneys, spleen, liver, lungs, marrow, small intestine and abdominal smooth muscle.

Preliminary results showed a 6-9 hours half-disappearance time for the material used in the above mentioned partial exchange transfusions.

Although in the specification the utilization of human hemoglobin was illustrated, it should be understood that other hemoglobin sources, such as bovine or porcine may be conceived to be utilized.

## Claims

1. Hemoglobin-based blood substitute resulted from stroma-free hemoglobin, modified with pyridoxal-5'-phosphate and intramolecularly stabilized, followed by the polymerization to an extent of above 40%, under anaerobic conditions of the stabilized PLP-hemoglobin, characterized in that the intramolecular stabilization is effected by reaction with a haloester of a dicarboxylic acid, said blood substitute material possessing a hemoglobin concentration in the range of 12 to 10 g/100 ml, possessing a P₅₀ in the range of 25 to 30 torr (25 to 30 mm Hg), and possessing a colloid osmotic pressure in the range of 24 to 31 torr (24 to 31 mm Hg).

2. Hemoglobin-based blood substitute material according to claim 1, possessing a methemoglobin content in the range of 3% to 6% of the total hemoglobin.

3. Hemoglobin-based blood substitute according to claims 1 or 2, wherein the hemoglobin is derived from whole human blood, or packed human red blood cells.

4. Hemoglobin-based blood substitute according to claims 1,2 or 3, wherein the extent of polymerization is above 50%.

5. Hemoglobin-based blood substitute according to claim 4, wherein the extent of polymerization is in the range of 50% to 80%.

6. A method for obtaining hemoglobin-based blood substitute which comprises the steps of:
(a) producing stroma-free hemoglobin;
(b) pyridoxylation of the stroma-free hemoglobin with pyridoxal-5'-phosphate;
(c) intramolecular stabilization of the PLP-hemoglobin, and
(d) polymerization to an extent of above 40%, under anaerobic conditions, of the stabilized PLP-hemoglobin, using a polyfunctional agent having groups capable of reacting with linkable sites on the hemoglobin molecule, characterised in that in step c) the intramolecular stabilization of the PLP-hemoglobin is effected by reaction with haloester of dicarboxylic acid.

7. A method according to claim 6 wherein the polyfunctional agent used in step (d) contains a carbonyl group.

8. A method according to claim 7, wherein said polyfunctional group is glutaraldehyde or a precursor thereof.

9. A method according to claim 7 or 8, wherein the molar ratio between the polymerizing reagent and hemoglobin is between 1:1 to 30:1.

10. A method acording to claims 6, 7 or 8 wherein the stabilized, PLP-hemoglobin solution used in the polymerization has a hemoglobin concentration in the range of between 7% to 16% (w/v).

11. A method according to any one of claims 6 to 10, wherein the polymerized, stabilized PLP-hemoglobin is quenched in a quencher solution.

12. A method according to claim 11, wherein said quencher is selected from the group consisting of amines, bisulphites, alcohols, isocyanates and mercaptans.

13. A method according to claim 12, wherein amine is selected from the group consisting of ethanolamine and lysine.

14. A method according to any one of claims 6 to 13, wherein the polymerized, stabilized PLP-hemoglobin, is reduced by a solution of sodium borohydride.

## Patentansprüche

1. Hämoglobin-basierender Blutersatz, der von stromafreiem Hämoglobin ausgeht, das mit Pyridoxal-5'-phosphat modifiziert ist und intramolekular stabilisiert ist, gefolgt von einer Polymerisation bis zu einem Grad von über 40% unter anaeroben Bedingungen des stabilisierten PLP-Hämoglobins, dadurch gekennzeichnet, daß die intramolekulare Stabilisierung durch Reaktion mit einem Haloester einer Dicarbonsäure bewirkt wird, wobei der Blutersatzstoff eine Hämoglobinkonzentration im Bereich von 12 bis 18g/100 ml besitzt, einen P₅₀ im Bereich von 25 bis 30 Torr (25 bis 30 mm Hg) besitzt und einen kolloidosmotischen Druck im Bereich von 24 bis 31 Torr (24 bis 31 mm Hg) besitzt.

2. Hämoglobin-basierender Blutersatz nach Anspruch 1, der einen Methämoglobingehalt im Bereich von 3 bis 6% des gesamten Hämoglobins besitzt.

3. Hämoglobin-basierender Blutersatz nach Anspruch 1 oder 2, wobei sich das Hämoglobin von vollständigem menschlichem Blut oder abgepackten menschlichen roten Blutzellen ableitet.

4. Hämoglobin-basierender Blutersatz nach den Ansprüchen 1, 2 oder 3, wobei der Polymerisationsgrad über 50% liegt.

5. Hämoglobin-basierender Blutersatz nach Anspruch 4, wobei der Polymerisationsgrad im Bereich von 50-80% liegt.

6. Verfahren zur Herstellung des Hämoglobin-basierenden Blutersatzes, das die folgenden Schritte umfaßt:
(a) Herstellung eines stromafreien Hämoglobins,
(b) Pyridoxylierung des stromafreien Hämoglobins mit Pyridoxal-5'-phoshat,
(c) intramolekulare Stabilisierung des PLP-Hämoglobins und
(d) Polymerisierung bis zu einem Grad von über 40% des stabilisierten PLP-Hämoglobins unter anaeroben Bedingungen unter Verwendung eines polyfunktionalen Agens mit Gruppen, die mit verknüpfbaren Stellen auf dem Hämoglobinmolekül reagieren können,
dadurch gekennzeichnet, daß im Schritt (c) die intramolekulare Stabilisierung des PLP-Hämoglobins durch eine Reaktion mit einem Haloester einer Dicarbonsäure bewirkt wird.

7. Verfahren nach Anspruch 6, wobei das polyfunktionale Agens, das im Schritt (d) eingesetzt wird, eine Carbonylgruppe enthält.

8. Verfahren nach Anspruch 7, wobei die polyfunktionale Gruppe Glutaraldehyd oder einen Vorläufer davon darstellt.

9. Verfahren nach Anspruch 7 oder 8, wobei das Molverhältnis zwischen dem polymerisierenden Reagens und dem Hämoglobin 1:1 bis 30:1 beträgt.

10. Verfahren nach den Ansprüchen 6, 7 oder 8, wobei die stabilisierte PLP-Hämoglobinlösung, die bei der Polymerisation verwendet wird, eine Hämoglobinkonzentration im Bereich von 7 bis 16% (Gew./Vol.) aufweist.

11. Verfahren nach einem der Ansprüche 6 bis 10, wobei das polymerisierte, stabilisierte PLP-Hämoglobin in einer Quenchlösung gequencht wird.

12. Verfahren nach Anspruch 11, wobei der Quencher aus der Gruppe ausgewählt ist, die aus Aminen, Bisulfiten, Alkoholen, Isocyanaten und Mercaptanen besteht.

13. Verfahren nach Anspruch 12, wobei das Amin aus der Gruppe ausgewählt ist, die aus Ethanolamin und Lysin besteht.

14. Verfahren nach einem der Ansprüche 6 bis 13, wobei das polymerisierte, stabilisierte PLP-Hämoglobin durch eine Natriumborhydridlösung reduziert wird.

## Revendications

1. Substitut sanguin à base d'hémoglobine, obtenu à partir d'une hémoglobine exempte de stroma, modifiée par du 5'-phosphate de pyridoxal et stabilisée intramoléculairement, avec ensuite polymérisation dans une proportion de plus de 40 %, la PLP-hémoglobine stabilisée étant en conditions anaérobies, caractérisé en ce que la stabilisation intramoléculaire est réalisée par réaction avec un halogénoester d'un acide dicarboxylique, ledit matériau substitut sanguin ayant une concentration en hémoglobine comprise dans l'intervalle allant de 12 à 10 g par 100 ml, possédant une P₅₀ comprise dans l'intervalle allant de 25 à 30 torr (25 à 30 mm Hg) et possédant une pression oncotique comprise dans l'intervalle allant de 24 à 31 torr (24 à 31 mm Hg).

2. Matériau substitut sanguin à base d'hémoglobine selon la revendication 1, ayant une concentration en méthémoglobine comprise dans l'intervalle allant de 3 % à 6% de l'hémoglobine totale.

3. Substitut sanguin à base d'hémoglobine selon les revendications 1 ou 2, dans lequel l'hémoglobine est dérivée de sang humain total, ou de globules rouges de sang humain concentrés.

4. Substitut sanguin à base d'hémoglobine selon les revendications 1, 2 ou 3, dans lequel le taux de polymérisation est supérieur à 50%.

5. Substitut sanguin à base d'hémoglobine selon la revendication 4, dans lequel le taux de polymérisation est compris dans l'intervalle allant de 50 % à 80 %.

6. Méthode pour obtenir un substitut sanguin à base d'hémoglobine qui comprend les étapes de :
(a) production d'hémoglobine exempte de stroma ;
(b) pyridoxylation de l'hémoglobine exempte de stroma par le 5'-phosphate de pyridoxal ;
(c) stabilisation intramoléculaire de la PLP-hémoglobine et
(d) polymérisation dans une proportion de plus de 40 %, en conditions anaérobies, de la PLP-hémoglobine stabilisée a utilisant un agent polyfonctionnel ayant des groupes capables de réagir avec des sites de la molécule d'hémoglobine pouvant contracter des liaisons, caractérisée en ce que dans l'étape (c), la stabilisation intramoléculaire est réalisée par réaction avec un halogénoester d'un acide dicarboxylique.

7. Méthode selon la revendication 6, dans laquelle l'agent polyfonctionnel utilise dans l'étape (d) contient un groupe carbonyle.

8. Méthode selon la revendication 7, dans laquelle ledit groupe polyfonctionnel est le glutaraldéhyde ou un de ses précurseurs.

9. Méthode selon la revendication 7 ou 8, dans laquelle le rapport molaire entre le réactif de polymérisation et l'hémoglobine est situé entre 1:1 et 30:1.

10. Méthode selon les revendications 6, 7 ou 8 dans laquelle la solution de PLP-hémoglobine stabilisée utilisée dans la polymérisation a une concentration en hémoglobine comprise dans l'intervalle allant de 7 à 16 % (masse/volume).

11. Méthode selon une quelconque des revendications 6 à 10, dans laquelle la PLP-hémoglobine stabilisée polymérisée est fixée dans une solution arrêtant les réactions.

12. Méthode selon la revendication 11, dans laquelle ledit produit arrêtant les réactions est sélectionné dans le groupe comprenant des amines, des bisulfites, des alcools, des isocyanates et des mercaptans.

13. Méthode selon la revendication 12, dans laquelle l'amine est sélectionnée dans le groupe comprenant l'éthanolamine et la lysine.

14. Méthode selon une quelconque des revendications 6 à 13, dans laquelle la PLP-hémoglobine stabilisée polymérisée est réduite par une solution de borohydrure de sodium.
